# EUROPEAN PATENT SPECIFICATION

(11) **EP 0 471 177 B1**
(45) Date of publication and mention of the grant of the patent: **04.10.1995**
(21) Application number: 91110919.7
(22) Date of filing: 02.07.1991
(51) Int. Cl.: A61K 39/10, A61K 39/385

(54) **Filamentous hemagglutinin of bordetella pertussis as a carrier molecule for conjugate vaccines**
Faser-Hemagglutinin von Bordetella pertussis als Träger für konjugierten Impfstoff
Hemagglutinine filamenteuse de Bordetella pertussis à titre de molécules porteuses pour vaccins conjugués

(30) Priority: 13.08.1990 US 565161
(43) Date of publication of application: 19.02.1992
(73) Proprietor: AMERICAN CYANAMID COMPANY, Wayne, NJ 07470-8426 (US)
(72) Inventor: Kimura, Alan, Miami, FL 33178 (US); Dick, William Edwin JR., Webster, NY (US); Cowell, James Leo, Fairport, NY 14450 (US)
(74) Representative: Wächtershäuser, Günter, Prof. Dr.

(56) References cited:
- EP-A- 0 080 021
- EP-A- 0 471 954
- FR-A- 2 407 000
- US-A- 4 761 283

## Description

### Background of the Invention

Enhanced immunogenic responses in infants to antigens including those containing carbohydrates have been observed where such antigens have been coupled to carrier proteins to form conjugate vaccines.

A variety of proteins, including pili, outer membrane proteins, secreted toxins of pathogenic bacteria and nontoxic proteins, including cross-reacting materials, such as CRMs of toxins (cross-reacting materials antigenically similar to bacterial toxins) have been used as carrier proteins for carbohydrate antigens. Of these proteins, tetanus toxoid and diphtheria toxoid have been the most widely employed as carriers for bacterial capsular polysaccharide (CPS) and lipopolysaccharide (LPS) antigens. See, for example, Dick and Beurret, "Conjugate Vaccines," Ed. Karger, p. 48-114 (1989); Goren, J. Immun., 98: 901-913 (1967) and Claesson, et al., J. Pediat., 112: 695-702 (1988).

Toxoiding of diphtheria and tetanus toxins blocks or modifies some of the reactive sites of amino acids within the protein chains, which can reduce immunogenicity and steric accessibility of the carrier. Consequently, successful use of toxoids typically requires that antigen loading be kept lower than levels obtained with native proteins. Linker arms can be employed to reach otherwise inaccessible sites. Beuvery, et al., Vaccine 1, 31-36 (1983); Cryz, et al., J. Infect. Dis., 154: 682-688 (1986). However, anticarrier response can fall sharply as the number of conjugation sites increase, thereby possibly inactivating T- and B-cell epitopes. Schneerson, et al., Prog. Allergy, 33: 144-158 (Karger, Basel 1983); Schneerson, et al., Prog. Clin. Biol. Res., 47: 77-94 (1980). Moreover, concerns persist about hypersensitivity to carrier proteins and suppression of anticarbohydrate response when pre-existing anticarrier titers are high. Snippe, et al., Infect. Immunity, 40: 856-861 (1983); Cryz, et al., supra. This situation may exist with the diphtheria and tetanus toxoids.

"Nonsense" proteins, or conjugates utilizing proteins from sources other than human pathogenic bacteria, are useful for developing conjugation methodologies and purification techniques. However, their ability to enhance anticarbohydrate responses varies widely. For example, proteins derived from mammaliam sources (e.g., bovine serum albumin (BSA), bovine gamma globulin (BGG), etc.), are used as carriers in research. They are, however, generally unsuitable for clinical applications.

Therefore, a need exists for a nontoxic, effective protein carrier for antigens containing carbohydrates which overcomes or minimizes the above-mentioned problems.

### Summary of the Invention

This invention pertains to immunogenic conjugates and vaccine compositions containing the immunogenic conjugate. The conjugates comprise an antigen bound to a filamentous hemagglutinin (FHA) of Bordetella pertussis, or portion thereof. The invention also pertains to a vaccine composition comprising the conjugate and a pharmaceutically acceptable vehicle. The invention further pertains to a method for eliciting a protective immune response which comprises administering to a vertebrate an immunogenic amount of a vaccine composition comprising the immunogenic conjugate of the present invention in a pharmaceutically acceptable vehicle and an optional adjuvant.

FHA has several advantages as a carrier protein. It is nontoxic and has a relatively high molecular weight (200,000 kDa), and after conjugation with antigens, FHA retains both T-cell and B-cell epitopes, which are important for function as an immunologically active conjugate.

### Detailed Description of the Invention

The immunogenic conjugates of this invention comprise an antigen bound to a filamentous hemagglutinin (FHA) of Bordetella pertussis, an immunologically active portion thereof or an immunologically cross-reactive mutant form. The conjugation of the antigen to FHA provides an immunogenic conjugate which can induce or enhance an immune response to the antigen.

FHA of Bordetella pertussis can be produced by a method described by Sato et al., Separation and Purification of the Hemagglutinins from Bordetella pertussis, Infect. Immun, 41: 313-320 (1983). FHA can also be produced by recombinant DNA technology.

The antigen can be derived from any source to which an immunogenic response is desired. The antigen need not itself be immunogenic, but can be of a type which becomes immunogenic by virtue of conjugation to FHA of Bordetella pertussis.

The antigen can be, for example, a carbohydrate (such as an oligosaccharide or polysaccharide), protein, peptide, peptidoglycan or glycopeptide. Carbohydrate-containing antigens of interest include: bacterial capsular polymers; lipopolysaccharide (LPS) or lipooligosaccharide (LOS) components of gram negative bacteria including oligosaccharides derived from LPS or LOS; auto-immunity related antigens such as those associated with rheumatoid arthritis and lupus erythematosus; allergens; tumor-associated antigens; and bacterial cell wall components such as peptidoglycans and fragments thereof.

Bacterial capsular polymers, oligomers and fragments thereof are among the groups of antigens which have potential to be effectively employed in a vaccine but which are only weakly immunogenic in young humans. As used in this application, the term "capsular polymers" refers to sugar-containing polymers, such as polymers of sugars, sugar acids, amino sugars, and sugar phosphates. These "capsular polymers" are frequently referred to in the medical literature as "capsular polysaccharides" though they may contain linkages other than glycosidic linkages and constituents other than sugars such as those listed above.

The capsular polymers (CP) can be derived from many different types of bacteria. These types include Haemophilus influenzae, Streptococcus species including pneumoniae (particularly serotypes 1, 4, 5, 6A, 6B, 9V, 14, 18C, 19F, and 23F), pyogenes and Neisseria agalactiae, Neisseria meningitidis (such as serogroups A, B and C), Klebsiella pneumoniae, Pseudomonas aeruginosa and Staphylococcus aureus. A preferred carbohydrate antigen is the oligosaccharide of Haemophilis influenzae type b, polyribosylribitolphosphate.

Other bacterial antigens of interest are those associated with the human bacterial pathogens including, for example, typable and untypable Escherichia coli, Branhamella catarrhalis, Vibrio cholerae, Bordetella pertussis, Bordetella bronchiseptica, Bordetella parapertussis, Corynebacteria diphtheriae, Neisseria gonorrhoeae, Neisseria meningitidis, Pseudomonas aeruginosa, Staphylococcus aureus, Chlamydia trachomatis and Clostridium tetani. Some specific bacterial antigens include bacterial outer membrane proteins (e.g. from Haemophilus influenzae, Neisseria meningitidis or Branhamella catarrhalis).

Viral antigens from pathogenic viruses can also be used with the present invention, including human immunodeficiency virus (types I and II), human T lymphocyte virus (types I, II and III), respiratory syncytial virus, hepatitis A, hepatitis B, hepatitis C, non-A and non-B hepatitis virus, herpes simplex virus (types I and II), cytomegalovirus, influenza virus, parainfluenza virus, poliovirus, rotavirus, coronavirus, rubella virus, measles virus, varicella, Epstein Barr virus, adenovirus, papilloma virus and yellow fever virus.

Several specific viral antigens of these pathogenic viruses include the F protein (especially antigens containing the F peptide 283-315, described in WO89/02935 entitled "Respiratory Syncytial Virus: Vaccines and Diagnostic Assays" by Paradiso, P. et at.) and the N and G proteins of respiratory syncytial virus (RSV), VP4 (previously known as VP3), VP6 and VP7 polypeptides of rotavirus, envelope glycoproteins of human immunodeficiency virus and the surface and presurface antigens of hepatitis B and herpes glycoproteins B and D.

Examples of parasitic antigens include but are not limited to Plasmodium spp. (especially circumsporozoite proteins), Eimeria spp., Schistosoma spp., Trypanosoma spp., Babesia spp., Leishmania spp., Cryptosporidia spp., Toxoplasma spp. and Pneumocysitis spp. Antigens derived from yeast and fungi can also be used with the present invention. Fungal antigens can be those derived from fungi including but not limited to Candida spp. (especially those from albicans), Cryptococcus spp. (especially neoformans), Blastomyces spp. (e.g., dermatitidis), Histoplasma spp. (especially capsulatum), Coccidroides spp. (especially immitis), Paracoccidroides spp. (especially brasiliensis) and Aspergillus spp. Further, tumor-associated or tumor-specific antigens, such as protooncogene or oncogene products, are suitable for use with the present invention.

The conjugates of this invention can be prepared by any of the biologically compatible methods known in the art for coupling of antigens to carriers. The method of coupling is most preferably covalent coupling whereby the antigen is bound directly to the FHA of Bordetella pertussis. However, other means by which the antigen is conjugated to the FHA of Bordetella pertussis is included within the scope of the invention. Many such methods are currently available for coupling of antigens to protein and peptide carriers. See, e.g., EP-A-89 908 669.8, entitled T-Cell Eptitope as Carrier Molecules for Conjugate Vaccines, by Bixler, Pillai and Insel, filed January 31, 1989, the teachings of which are incorporated herein by reference. A particularly preferred method for coupling the antigen to the FHA carrier is by reductive amination which has been described by Anderson, P.W., U.S. Patent No. 4,673,573, issued June 16, 1987, and U.S. Patent No. 4,761,283, issued August 2, 1988.

The conjugates of this invention can be used to elicit an immune response to an antigen or saccharide in a warm-blooded animal (such as a mammalian host). The method comprises administering to the animal, an immunologically effective dose of the conjugate comprising an antigen bound to FHA.

Vaccine compositions comprise the conjugates in a pharmaceutically acceptable vehicle, such as physiological saline, or ethanol polyols (such as glycerol or propylene glycol). The vaccine compositions may optionally comprise adjuvants, such as vegetable oils or emulsions thereof, surface active substances, e.g., hexadecylamine, octadecyl amino acid esters, octadecylamine, lysolecithin, dimethyl-dioctadecylammonium bromide, N,N-dioctadecyl-N'-N'bis (2-hydroxyethyl-propane diamine), methoxyhexadecylglycerol, and pluronic polyols; polyamines, e.g., pyran, dextransulfate, poly IC, carbopol; peptides, e.g., muramyl dipeptide, dimethylglycine, tuftsin; immune stimulating complexes; oil emulsions; and mineral gels. The conjugates of this invention may also be incorporated into liposomes or ISCOMS (immunostimulating complexes). Supplementary active ingredients may also be employed. The conjugate can also be adsorbed onto a mineral suspension, such as alum, i.e., aluminum hydroxide or aluminum phosphate to further modulate the protective immune response to the antigen.

The vaccines can be administered to a human or animal in a variety of ways. These include intradermal, transdermal (such as by slow release polymers), intramuscular, intraperitoneal, intravenous, subcutaneous, oral and intranasal routes of administration. The amount of conjugate employed in such vaccine will vary depending upon the identity of the antigen employed. Adjustment and manipulation of established dosage ranges used with traditional carrier conjugates for adaptation to the present conjugate vaccines is well within the ability of those skilled in the art. The conjugates of the present invention are intended for use in the treatment of both immature and adult warm-blooded animals, and in particular humans. In addition, the use of the present methods and conjugates is not limited to prophylactic application; therapeutic application are also contemplated (e.g., AIDS therapy and post-exposure prophylaxis).

A vaccine composition which can be useful in the vaccination against meningitis caused by Haemophilus will comprise the oligomer polyribosylribitolphosphate (PRP) of Haemophilus influenzae type b conjugated to FHA of Bordetella pertussis. Bacterial meningitis in the United States is most commonly caused by H. influenzae type b.

The immunogenic conjugates of the invention can be admixed with an antigen from the same or different organism in a pharmaceutically acceptable vehicle and an optional adjuvant to produce a co-vaccine which can be used to elicit an immune response to both the conjugated antigen and the admixed antigen.

Suitable antigens for use in the co-vaccine compositions of the invention include the particulate antigens such as bacteria, viruses and microcomponents of cells. In addition, the antigens described above can be used.

FHA of Bordetella pertussis can help evoke a protective immune response against marginally or non-immunogenic antigens when conjugated to FHA. In this manner, vaccine compositions containing fragments of larger proteins, synthetic antigens or products of recombinant DNA technology may be made more potent as conjugates of the present invention.

It is to be understood from the above discussion, that the use of the term antigen is meant to imply either the whole antigen or one of its determinants, and is also meant to encompass hapten molecules which could benefit by an increase in the immune response due to the presence of the conjugates of the present invention. The foregoing list of antigens is for exemplary purposes only. Additional antigens which can be used in the co-vaccine compositions of the present invention are readily ascertained by one skilled in the art.

The invention is further illustrated by the following non-limiting Exemplification.

### EXEMPLIFICATION

### FHA Purification

FHA was purified from culture supernatants of Bordetella pertussis strain Tohama I using the procedure described by Sato et al. , supra. FHA thereby obtained was then dialyzed against deionized water to remove residual buffer, using a Pierce microdialyzer fitted with an 8K membrane. The process completely precipitated the previously soluble FHA, producing a loose gel of protein.

### Preparation of Oligosaccharide

Polyribosylribitolphosphate (PRP) oligosaccharides (HbO) were prepared as described by Anderson, et al. , J. Immunol., 137: 1181-1186 (1983).

### Conjugation of PRP Oligosaccharides to FHA

A slurry containing 2.1 mg of FHA was mixed with 6.5 mg of Haemophilus influenzae type b (HbO) in a dilute, aqueous solution in a 15 ml polypropylene tube. The mixture was then lyophilized and redispersed with stirring in 1 ml DMSO. After 8 hours at 35°C, 2 mg (100 µl) of a sodium cyanoborohydride stock solution in DMSO was added to initiate reductive amination to thereby conjugate the FHA and HbO. The reaction mixture was stirred 48 hours longer at 35° C, before adding a 20 µl portion of water. The mixture was stirred for 24 hours, maintaining temperature, before adding 1 mg of NaBH₄ to cap the reaction. After a final 24 hours at 25° C, the mixture was lyophilized.

The residue was dispersed in a 1 ml portion of water at 4°C and centrifuged. The pellet was washed by two 1 ml resuspension and centrifugation cycles. Suspension of the washed pellet in 0.9% saline gave a slurry that tested strongly positive for both protein and carbohydrate components. These combined pellet supernatants were combined, lyophilized, resuspended in water, and purified on a BioGel® P-100 column packed and eluted with water. Orcinol and Lowry as says were used to locate fractions containing both FHA and HbO, and fractions containing free HbO. The pooled conjugate fractions were lyophilized, resuspended in 0,9% saline, and recombined with the original washed pellet.

### Immunogenicity of FHA-PRP conjugate vaccines

The oligosaccharide-FHA conjugate vaccine was adsorbed to aluminum phosphate adjuvant and two subcutaneous injections of 35 µg protein were given to Swiss-Webster mice at four week intervals. Control mice received either aluminum phosphate alone, aluminum phosphate mixed with oligosaccharide, or aluminim phosphate mixed with oligosaccharide and FHA. The mice were bled at week 0, 3, 4 and 6. The sera were assayed for anti-PRP antibodies by radioimmunoassay and for anti-FHA antibodies by ELISA.

### Results

The anti-PRP antibody responses and anti-FHA antibody are shown in Tables 1 and 2, respectively. As can be seen in Table 1, control mice had no detectable antibody response to PRP. In contrast, mice injected with the oligosaccharide-FHA conjugate vaccine had 2.3 µg/ml and 5.18 µg/ml of anti-PRP antibody at 3 and 4 weeks post-immunization, respectively. After the second immunization at week 4, the anti-PRP antibody response was boosted to 26.53 µg/ml at week 6. As can be seen in Table 2, mice injected with the oligosaccharide-FHA conjugate also contributed an anti-FHA antibody response. At weeks 3, 4 and 6, the mice had anti-FHA antibody titers of: 3,864, 5,696 and 355,142, respectively.

### Discussion

In the experiment presented here, PRP oligosaccharides conjugated to FHA induced substantial antibody responses to PRP which was boosted after the second injection. This finding provides evidence that FHA is functioning as a T-cell dependent carrier for the oligosaccharides. The conjugated FHA also retained B-cell epitopes as evidenced by the antibody response detected by ELISA against FHA.

**TABLE 1**

| Immunogenicity of PRP Oligosaccharide - FHA conjugate vaccine in Swiss-Webster mice. | | | | |
|---|---|---|---|---|
| Vaccine^{a} (dose per mouse) | Anti-PRP polysaccharide antibody (µg/ml serum)^{b} | | | |
| | Week | | | |
| | 0 | 3 | 4 | 6 |
| 1. Control | | | | |
| Al(PO₄) (100µg) | <0.10 | <0.10 | <0.10 | <0.10 |

| 2. Control | | | | |
|---|---|---|---|---|
| Al(PO₄) (100µg) | <0.10 | <0.10 | <0.10 | <0.10 |
| PRP oligosaccharide (70µg) | | | | |

| 3. Control | | | | |
|---|---|---|---|---|
| Al(PO₄) (100µg) | <0.10 | <0.10 | <0.10 | <0.10 |
| PRP oligosaccharide (70µg) | | | | |
| FHA (35µg) | | | | |
| 4. PRP oligosaccharide - FHA conjugate (35µg protein) | <0.10 | 2.30 | 5.18 | 26.53 |

| | | | | |
|---|---|---|---|---|
| ^{a}Vaccines were administered by the subcutaneous route (0.2 ml) at week 0 and 4. Control vaccines consisted of either Al(PO₄) alone (vaccine number 1), Al(PO₄) mixed with PRP oligosaccharide (vaccine number 2), or Al(PO₄) mixed with PRP oligosaccharide and FHA (vaccine number 3). | | | | |
| ^{b}Antibodies to the PRP capsular polysaccharide of *H*. *influenzae* type b were measured by radioimmunoassay. | | | | |

**TABLE 2**

| Anti-FHA antibody response to PRP oligosaccharide-FHA conjugate vaccine in Swiss-Webster mice. | | | | |
|---|---|---|---|---|
| Vaccine^{a} (dose per mouse) | Anti-FHA antibody response^{b} | | | |
| | Week | | | |
| | 0 | 3 | 4 | 6 |
| 1. Control | | | | |
| AlPO₄ (100µg) | <50 | <50 | <50 | <50 |

| 2. Control | | | | |
|---|---|---|---|---|
| AlPO₄ (100µg) | <50 | <50 | <50 | <50 |
| PRP oligosaccharide (70µg) | | | | |

| 3. Control | | | | |
|---|---|---|---|---|
| AlPO₄ (100µg) | <50 | 389,070 | 921,111 | 1,214,909 |
| PRP oligosaccharide (70µg) | | | | |
| FHA (35µg) | | | | |
| 4. PRP oligosaccharide-FHA conjugate (35µg protein) | <50 | 3,864 | 5,696 | 355,142 |

| | | | | |
|---|---|---|---|---|
| ^{a}Vaccines were administered by the subcutaneous route (0.2ml) at week 0 and 4. Control vaccines consisted of either AlPO₄ alone (vaccine number 1), AlPO₄ mixed with PRP oligosaccharide (vaccine number 2), or AlPO₄ mixed with PRP oligosaccharide and FHA (vaccine number 3). | | | | |
| ^{b}Antibodies to B. pertussis FHA were measured by ELISA. Values are the reciprocol of the dilution giving an absorbance of 0.1 calculated from a linear plot of log of absorbance versus log of dilution. | | | | |

## Claims

1. An immunogenic conjugate, comprising an antigen coupled to a filamentous hemagglutinin of Bordetella pertussis or an immunologically active portion thereof, or to an immunologically cross-reactive mutant filamentous hemagglutinin of Bordetella pertussis or portion thereof.

2. An immunogenic conjugate of Claim 1, wherein the antigen is a carbohydrate.

3. An immunogenic conjugate of Claim 2, wherein the carbohydrate antigen is a bacterial capsular oligosaccharide or polysaccharide, or a fragment of a capsular oligosaccharide or polysaccharide.

4. An immunogenic conjugate of Claim 3, wherein the capsular oligosaccharide or polysaccharide is derived from a bacterium selected from the group consisting of Haemophilus influenzae, Streptococcus pneumoniae, Neisseria meningitidis, Klebsiella pneumoniae, Pseudomonas aeruginosa and Staphylococcus aureus.

5. An immunogenic conjugate of Claim 2, wherein the carbohydrate antigen is a polyribosylribitolphosphate.

6. An immunogenic conjugate of Claim 1, which is the product of reductive amination of the antigen and the filamentous hemagglutinin.

7. An immunogenic conjugate, comprising polyribosylribitolphosphate coupled to a filamentous hemagglutinin of Bordetella pertussis or an immunologically active portion thereof, or to an immunologically cross-reactive mutant filamentous hemagglutinin of Bordetella pertussis or portion thereof.

8. A vaccine composition, comprising an immunogenic conjugate which is an antigen coupled to a filamentous hemagglutinin of Bordetella pertussis or an immunologically active portion thereof, or to an immunologically cross-reactive mutant filamentous hemagglutinin of Bordetella pertussis or portion thereof, and a pharmaceutically acceptable vehicle.

9. An immunogenic conjugate of Claim 8 wherein the antigen is a carbohydrate antigen.

10. A vaccine composition of Claim 9, wherein the carbohydrate antigen is polyribosylribitolphosphate.

## Patentansprüche

1. Immunogenes Konjugat, umfassend ein Antigen, das an ein faseriges Hämagglutinin von Bordetella pertussis oder an einen immunologisch aktiven Teil desselben oder an ein immunologisch kreuzreaktives mutiertes faseriges Hämagglutinin von Bordetella pertussis oder an einen Teil desselben gekuppelt ist.

2. Immunogenes Konjugat nach Anspruch 1, in dem das Antigen ein Kohlehydrat ist.

3. Immunogenes Konjugat nach Anspruch 2, in dem das Kohlehydrat-Antigen ein bakterielles kapsuläres Oligosaccharid oder Polysaccharid oder ein Fragment eines kapsulären Oligosaccharids oder Polysaccharids ist.

4. Immunogenes Konjugat nach Anspruch 3, in dem das kapsuläre Oligosaccharid oder Polysaccharid von einem Bakterium abstammt, das aus der Gruppe ausgewählt ist, die aus Haemophilus influenzae, Streptococcus pneumoniae, Neisseria meningitidis, Klebsiella pneumoniae, Pseudomonas aeruginosa und Staphylococcus aureus besteht.

5. Immunogenes Konjugat nach Anspruch 2, in dem das Kohlehydrat-Antigen ein Polyribosylribitphosphat ist.

6. Immunogenes Konjugat nach Anspruch 1, das das Produkt einer reduktiven Aminierung des Antigens und des faserigen Hämagglutinins ist.

7. Immunogenes Konjugat, umfassend Polyribosylribitphosphat, das an ein faseriges Hämagglutinin von Bordetella pertussis oder einen immunologisch aktiven Teil desselben oder an ein immunologisch kreuzreaktives mutiertes faseriges Hämagglutinin von Bordetella pertussis oder an einen Teil derselben gekuppelt ist.

8. Impfstoffzusammensetzung, umfassend ein immunogenes Konjugat, das ein Antigen ist, welches an ein faseriges Hämagglutinin von Bordetella pertussis oder an einen immunologisch aktiven Teil desselben oder an ein immunologisch kreuzreaktives mutiertes faseriges Hämagglutinin von Bordetella pertussis oder an einen Teil desselben gebunden ist, und ein pharmazeutisch annehmbares Vehikel.

9. Immunogenes Konjugat nach Anspruch 8, in dem das Antigen ein Kohlehydrat-Antigen ist.

10. Impfstoffzusammensetzung nach Anspruch 9, in der das Kohlehydrat-Antigen Polyribosylribitphosphat ist.

## Revendications

1. Conjugué immunogène comprenant un antigène couplé à une hémagglutinine filamenteuse de Bordetella pertussis ou une partie immunologiquement active de cette dernière ou à une hémagglutinine filamenteuse mutante à réaction immunologique croisée de Bordetella pertussis ou une partie de cette dernière.

2. Conjugué immunogène selon la revendication 1, dans lequel l'antigène est un saccharide.

3. Conjugué immunogène selon la revendication 2, dans lequel l'antigène saccharidique est un oligosaccharide ou un polysaccharide capsulaire bactérien ou un fragment d'un oligosaccharide ou d'un polysaccharide capsulaire.

4. Conjugué immunogène selon la revendication 3, dans lequel l'oligosaccharide ou le polysaccharide capsulaire provient d'une bactérie sélectionnée dans le coupe constitué par Haemophilus influenzae, Streptoccus pneumoniae, Neisseria meningitidis, Klehsiella pneumoniae, Pseudomonas aeruginosa et Staphylococcus aureus.

5. Conjugué immunogène selon la revendication 2, dans lequel l'antigène saccharidique est du polyribosylribitol phosphate.

6. Conjugué immunogène selon la revendication 1, lequel est le produit de l'amination réductrice de l'antigène et de l'hémagglutinine filamenteuse.

7. Conjugué immunogène, comprenant du polyribosylribitol phosphate couplé à une hémagglutinine filamenteuse de Bordetella pertussis ou à une partie immunologiquement active de cette dernière ou à une hémagglutinine filamenteuse mutante à réaction immunologique croisée de Bordetella pertussis ou une partie de cette dernière.

8. Composition vaccinale comprenant un conjugué immunogène qui est un antigène couplé à une hémagglutinine filamenteuse de Bordetella pertussis ou une partie immunologiquement active de cette dernière ou à une hémagglutinine filamenteuse mutante à réaction immunologique croisée de Bordetella pertussis ou à une partie de cette dernière et un véhicule pharmaceutiquement acceptable.

9. Conjugué immunogène selon la revendication 8, dans lequel l'antigène est un antigène saccharidique.

10. Composition vaccinale selon la revendication 9, dans laquelle l'antigène saccharidique est du polyribosylribitol phosphate.
